## Europäisches Patentamt

## European Patent Office

## Office européen des brevets

(19)

(11) Veröffentlichungsnummer: **0 086 880**
**B1**

(12) **EUROPÄISCHE PATENTSCHRIFT**

(45) Veröffentlichungstag der Patentschrift:
19.02.86

(21) Anmeldenummer: **82110499.9**

(22) Anmeldetag: **13.11.82**

(51) Int. Cl.⁴: **A 61 F 2/30**

(54) Den Schaft einer in einen Röhrenknochen einsetzbaren Endoprothese zentrierende Halterung.

(30) Priorität: **23.02.82 CH 1093/82**

(43) Veröffentlichungstag der Anmeldung:
**31.08.83 Patentblatt 83/35**

(45) Bekanntmachung des Hinweises auf die Patenterteilung:
**19.02.86 Patentblatt 86/8**

(84) Benannte Vertragsstaaten:
**AT BE DE FR GB IT NL**

(56) Entgegenhaltungen:
**EP - A - 0 010 527**
**EP - A - 0 058 744**
**CH - A - 542 624**
**DE - A - 1 961 531**
**DE - B - 2 247 560**
**DE - B - 2 338 136**
**US - A - 4 011 602**

(73) Patentinhaber: **GEBRÜDER SULZER AKTIENGESELLSCHAFT**, Zürcherstrasse 9,
**CH-8401 Winterthur (CH)**

(72) Erfinder: **Frey, Otto, Wallrütistrasse 56,**
**CH-8404 Winterthur (CH)**

(74) Vertreter: **Dipl.-Ing. H. Marsch Dipl.-Ing. K. Sparing Dipl.-Phys.Dr. W.H. Röhl Patentanwälte,**
**Rethelstrasse 123, D-4000 Düsseldorf (DE)**

## Beschreibung

Die Erfindung betrifft eine den Schaft einer in einen Röhrenknochen einsetzbaren Endoprothese zentrierende Halterung mit einem Führungstück für den mindestens im Bereich der Führung im Querschnitt invarianten Schaft, die als ein an dem Schaft angepasster Gleit-Pass-Sitz ausgebildet ist, und mit einem das Führungsstück im Knochen fixierenden aufspreizbaren Verankerungselement, sowie mit einem Spreizkörper, wobei der Aussenmantel des Spreizkörpers und der Innenhohlraum des Verankerungselementes mindestens über einen Teil ihrer axialen Länge von je einem in gleicher Richtung verlaufenden Konus begrenzt sind, auf denen sich der Spreizkörper und das Verankerungselement gegenseitig abstützen und das Aufspreizen des Verankerungselementes bewirken, wenn der Spreizkörper in dem verankerungselement in Richtung auf dessen Konus axial verstellt wird.

Verankerungsschäfte von Endoprothesen, beispielsweise von Kniegelenkprothesen, sollten in vielen Fällen so im Knochen gelagert werden, dass sie in axialer Richtung verschiebbar, in radialer Richtung jedoch zentriert sind. Zusätzlich tritt dabei häufig die Komplikation auf, dass die Abstützung des Schaftes, die im allgemeinen auf dem kortikalen Gewebe des Knochens erfolgt, in einem sich in axialer Richtung relativ stark verengenden oder in einem sich vom äusseren Ende her erweiternden Markhohlraum erfolgen muss. Dadurch wird die Fixierung einer zentrierenden Halterung im Knochen erschwert.

Aus der DE-B1-2 338 136 ist eine Halterung der genannten Art bekannt; diese ist für Kleingelenke, z.B. Fingergelenke, bestimmt. Sie besteht aus einem, an den Schaft im Gleis-Pass-Sitz angepassten Köcher, in den das Schaftende eingeschoben ist. In der axialen Verlängerung des Schaftendes ist das Verankerungselement mit Hilfe eines Spreizkörpers zur Fixierung im Knochen aufspreizbar. Diese Halterung hat den Nachteil, dass sie nicht an einer beliebigen Stelle der Schaftlänge im Knochen mit grosser Genauigkeit fixiert werden kann; darüberhinaus sind bei ihr Führungsweg und/oder Verankerungslänge sehr oft begrenzt und daher ungenügend.

Aufgabe der Erfindung ist es, eine Halterung für einen axial beweglichen, jedoch radial zentrierten Schaft zu schaffen, die selbst in jedem beliebigen Bereich von im Querschnitt etwa konstanten oder sich axial erweiternden bzw. verengenden Markhohlräumen sicher fixierbar ist und darüberhinaus ausreichend lange Führungswege und Verankerungslänge aufweist.

Gemäss der vorliegenden Erfindung wird diese Aufgabe dadurch gelöst, dass das Führungsstück als Spreizkörper ausgebildet ist und ein Aussengewinde aufweist, das mit einem Innengewinde des Verankerungselementes im Eingriff steht, und das Führungsstück zum Zwecke des Verankerns des Verankerungselementes in dieses eingeschraubt wird.

Der Schaftquerschnitt kann beliebig — d.h. beispielsweise kreisförmig, elliptisch oder oval — ausgebildet sein. Die Verlegung der Führung in den Spreizkörper ermöglicht, dass der Schaft die Halterung vollständig durchsetzt, so dass diese in jedem beliebigen axialen Bereich des Röhrenknochens, in dessen Markhohlraum das Verankerungselement eingeführt, an einer gewünschten Stelle festgehalten und mit Hilfe des eindringenden Spreizkörpers aufgespreizt und fixiert wird. Die erforderliche Genauigkeit, mit der die Halterung in einem bestimmten axialen Bereich des Knochens fixiert sein soll, wird dadurch erreicht, dass der Spreizkörper in das Verankerungselement eingeschraubt ist, da dann weniger die Gefahr besteht, dass das Verankerungselement infolge eines axialen Zuges oder Druckes verschoben wird; darüberhinaus kann eine Verschraubung, wenn notwendig, jederzeit wieder gelöst werden.

Die Fixierung der Halterung und/oder die Verankerung des Schaftes können sowohl völlig knochenzementfrei als auch in einem zusätzlich als Füllung des Markhohlraumes dienenden Zementbett erfolgen. Weiterhin können die eine Aufspreizung bewirkenden Konen an Spreizkörper und im Verankerungselement gleiche oder verschiedene Steigungen haben.

Um ein Aufspreizen des Verankerungselementes zu erleichtern, ist es vorteilhaft, dieses mit im wesentlichen in Achsrichtung verlaufenden Längsschlitzen zu versehen und/oder als einen verformbaren Hohlzylinder zu gestalten, der längs seines Umfanges wellenförmig ausgebildet ist.

Als Materialien für eine neue Halterung sind alle in der Implantat-Technik üblichen Werkstoffe geeignet; vorzugsweise wird für beide Einzelteile der Halterung Kunststoff, insbesondere Polyäthylen der Klassifikationen HDPE und UHMW verwendet. Weiterhin ist es möglich, die Oberflächen mindestens teilweise mit einer Beschichtung zu versehen, die beispielsweise die Reibung des Schaftes in dem Gleit-Pass-Sitz erniedrigt oder das Anwachsen von Gewebe fördert.

Im folgenden wird die Erfindung anhand eines Ausführungsbeispiels näher erläutert.

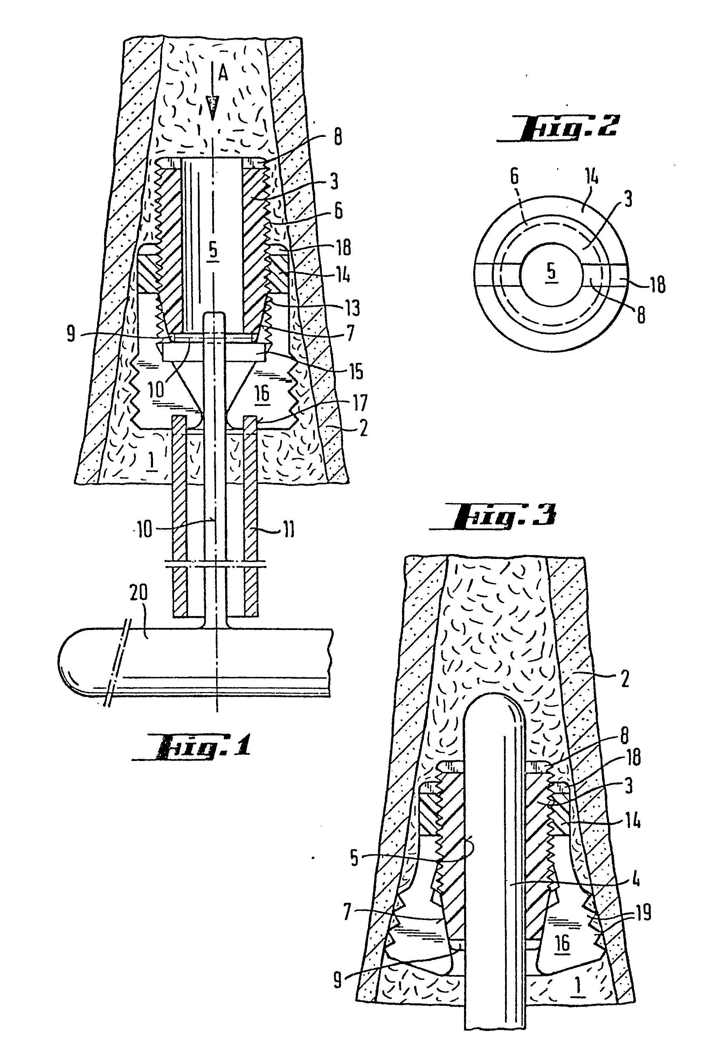

Fig. 1 zeigt schematisch im Längsschnitt die neue Halterung während des Einführens in den Markhohlraum eines Röhrenknochens;

Fig. 2 ist eine Aufsicht auf die Halterung nach Fig. 1 in Richtung des Pfeiles A;

Fig. 3 schliesslich gibt in gleicher Darstellung wie Fig. 1 die im Knochen fixierte Halterung wieder, in der ein Schaft gelagert ist.

Die Halterung umfasst ein zentrales Führungsstück 3 für einen Schaft 4 (Fig. 3), das als Spreizkörper ausgebildet ist. Ein als Führung dienender Hohlraum 5 des Führungsstückes 3 ist in Form und Abmessungen an dem im vorliegenden Beispiel kreiszylindrischen Querschnitt des Schaftes 4 angepasst, so dass der Schaft 4 in einem Gleit-Pass-Sitz im Führungsstück 3 axial beweglich geführt ist. Das Führungsstück 3 besitzt im wesentlichen eine kreiszylindrische Form, wobei der Aussenmantel des Zylinders etwa im unteren Drittel der axialen Länge des Führungsstückes 3 in einen Konus 7 übergeht. An seinen beiden Stirnseiten ist das Führungsstück 3 mit Schlitzen 8 und 9 für den Eingriff eines Werkzeugs 10 versehen.

Auf seinem Aussenmantel trägt das Führungsstück 3 ein Gewinde 6, welches in ein entsprechendes Gewinde 13 im Innenhohlraum 15 eines Verankerungselementes 14 einschraubbar ist. Der Innenhohlraum 15 verengt sich ähnlich wie der Aussenmantel des Führungsstückes 3 im unteren Drittel seiner axialen Länge konisch. Das Verankerungselement 14 ist in seiner Grundform ein Hohlzylinder, der über einen gewissen Bereich seiner axialen Höhe mit über den Umfang verteilten Längsschlitzen 16 versehen ist, die sein Aufspreizen erleichtern bzw. ermöglichen. Es hat an seinen Stirnseiten ebenfalls Vertiefungen bzw. Schlitze 17 und 18 für ein Werkzeug 11.

Im aufspreizbaren Bereich ist der Mantel des Verankerungselementes 14 mit einer Verzahnung 19 oder einzelnen noppenartigen Vorsprüngen versehen, durch die seine Haftung im kortikalen Knochengewebe 2 verbessert wird. Statt Verzahnung und Noppen können auch andere die Haftung fördernde Oberflächenstrukturen vorgesehen sein.

Zur Einführung in einen Markhohlraum 1 eines Knochens 2, der sich nach oben relativ stark verengt, werden das Führungsstück 3 und das Verankerungselement 14 an den beiden konzentrisch zueinander und zur Achse der Halterung angeordneten Werkzeugen 10 bzw. 11 befestigt, wobei beide Elemente 3 und 14 durch Einschrauben des Führungsstückes 3 in das Verankerungselement 14 leicht miteinander verbunden werden, sofern der operativ vorbereitete Markhohlraum eine genügend grosse Öffnung für ein gemeinsames Einführen der beiden Teile der Halterung hat. Im vorgesehenen axialen Abstand — der beispielsweise durch eine Markierung am Haltewerkzeug 11 für das Verankerungselement 14 festgelegt sein kann — vom Rand des geöffneten Knochens 2 hält man nun mit dem Werkzeug 11 das Verankerungselement 14 fest und schraubt mit Hilfe des Werkzeugs 10, das einen Handgriff 20 hat, das Führungsstück 3 soweit in das Verankerungselement 14 ein, dass dessen mit den Schlitzen 16 versehener Teil des Mantels mit einer ausreichenden Kraft gegen das kortikale Knochengewebe 2 gepresst wird, um eine sichere Fixierung der Halterung zu gewährleisten. Nach Lösen der Werkzeuge 10 und 11 von der Halterung kann der Schaft 4 in den Hohlraum 5 des Führungsstückes 3 eingeschoben werden (Fig. 3).

**Patentansprüche**

1. Den Schaft (4) einer in einen Röhrenknochen (1, 2) einsetzbaren Endoprothese zentrierende Halterung mit einem Führungsstück (3) für den mindestens im Bereich der Führung (5) im Querschnitt invarianten Schaft, die als ein an den Schaft angepasster Gleit-Pass-Sitz ausgebildet ist, und mit einem das Führungsstück (3) im Knochen (1, 2) fixierenden aufspreizbaren Verankerungselement (14), sowie mit einem Spreizkörper (3), wobei der Aussenmantel des Spreizkörper (3) und der Innenhohlraum des Verankerungselementes (14) mindestens über einen Teil ihrer axialen Länge von je einem in gleicher Richtung verlaufenden Konus begrenzt sind, auf denen sich der Spreizkörper (3) und das Verankerungselement (14) gegenseitig abstützen und das Aufspreizen des Verankerungselementes (14) bewirken, wenn der Spreizkörper (3) in dem Verankerungselement (14) in Richtung auf dessen Konus axial verstellt wird, dadurch gekennzeichnet, dass das Führungsstück (3) als Spreizkörper ausgebildet ist und ein Aussengewinde (6) aufweist, das mit einem Innengewinde (13) des Verankerungselementes (14) im Eingriff steht, und das Führungsstück (3) zum Zwecke des Verankerns des Verankerungselementes (14) in dieses eingeschraubt wird.

2. Halterung nach Anspruch 1, dadurch gekennzeichnet, dass das Verankerungselement (14) mit mindestens im wesentlichen in Achsrichtung verlaufenden Längsschlitzen (16) versehen ist.

3. Halterung nach Anspruch 1 oder 2, dadurch gekennzeichnet, dass das Verankerungselement (14) aus einem verformbaren Hohlzylinder besteht, der längs seines Umfangs wellenförmig ausgebildet ist.

**Revendications**

1. Support de centrage de la tige (4) d'une endoprothèse pouvant être implantée dans un os allongé (1, 2), comprenant une pièce (3) de guidage de la tige présentant une section constante au moins au voisinage de la zone de guidage (5) assurant une assise coulissante adaptée à cette tige; un élément d'ancrage (14) expansible, verrouillant la pièce de guidage (3) dans l'os (1, 2); ainsi qu'un corps d'expansion (3), l'enveloppe externe de ce corps d'expansion (3) et l'espace interne creux de l'élément d'ancrage (14) étant délimités, sur au moins une partie de leur longueur axiale, par un tronc de cône respectif s'étendant dans la même direction, sur lesquels le corp d'expansion (3) et l'élément d'ancrage (14) prennent mutuellement appui et provoquent l'expansion de l'élément d'ancrage (14) lorsque le corps d'expansion (3) est déplacé axialement, dans cet élément d'ancrage (14), en direction de son tronc de cône, caractérisé par le fait que la pièce de guidage (3) est réalisée sous la forme d'un corps d'expansion et présente un filetage externe (6) en prise avec un filetage interne (13) de l'élément d'ancrage (14), cette pièce de guidage (3) étant vissée dans l'élément d'ancrage (14) en vue de l'ancrage de ce dernier.

2. Support selon la revendication 1, caractérisé par le fait que l'élément d'ancrage (14) est muni de fentes longitudinales (16) s'étendant au moins sensiblement dans le sens axial.

3. Support selon la revendication 1 ou 2, caractérisé par le fait que l'élément d'ancrage (14) consiste en un cylindre creux déformable de réalisation ondulée le long de son pourtour.

**Claims**

1. A mounting centring the stem (4) of an endoprosthesis introducible into a long bone, (1, 2) the mounting having: a guide element (3) for the stem, the same being of invariable cross-section at least near the guide (5), the same being a snug fit on the stem; an expanding anchorage element (14) securing the guide element (3) in the bone (1, 2); and an expanding member (3), the outer generated surface thereof and the hollow interior of the anchorage element (14) being bounded over at least some of their axial lenght by in each case a cone extending in the same direction, on which cones the expanding member (3) and the anchorage element (14) bear on one another and expand the anchorage element (14) when the expanding member (3) is adjusted axially in the anchorage element (14) towards the cone thereof, characterized in that the guide element (3) is an expanding member and has an external screwthread (6) which engages with an internal screwthread (13) of the anchorage element (14), and the guide element (3) is screwed into the ancorage element (14) to anchor the same.

2. A mounting according to claim 1, characterized in that the anchorage element (14) is formed with longitudinal slots (16) extendings at least substantially axially.

3. A mounting according to claim 1 or 2, characterized in that the anchorage element (14) is in the form of a deformable hollow cylinder which is wavy along its circumference.

Fig. 1

Fig. 2

Fig. 3